# EUROPÄISCHE PATENTSCHRIFT

(11) **EP 3 638 116 B1**
(45) Veröffentlichungstag und Bekanntmachung des Hinweises auf die Patenterteilung: **24.03.2021**
(21) Anmeldenummer: 18729628.0
(22) Anmeldetag: 01.06.2018
(51) Int. Cl.: A61B 5/097, G01N 33/497

(54) **SYSTEM UND VERFAHREN FÜR EINE FREIGABE EINER MESSUNG VON ANALYTEN IN AUSATEMLUFT**
SYSTEM AND METHOD FOR ENABLING A MEASUREMENT OF ANALYTES IN EXHALED AIR
SYSTÈME ET PROCÉDÉ POUR UNE LIBÉRATION D'UNE MESURE D'ANALYTES DANS DE L'AIR EXPIRÉ

(30) Priorität: 13.06.2017 DE 102017209909
(43) Veröffentlichungstag der Anmeldung: 22.04.2020
(73) Patentinhaber: Robert Bosch GmbH, 70442 Stuttgart (DE)
(72) Erfinder: SCHNEIDER, Stefan, 71717 Beilstein (DE); THUERSAM, Markus, 71263 Weil Der Stadt (DE); BARTH, Frank, 71732 Tamm (DE); DOEHRING, Tom, 70329 Stuttgart (DE); MUELLER, Klaus, 70449 Stuttgart (DE)
(86) Internationale Anmeldenummer: PCT/EP2018/064474
(87) Internationale Veröffentlichungsnummer: WO 2018/228832

(56) Entgegenhaltungen:
- EP-A1- 0 328 415
- US-B1- 9 643 186

## Beschreibung

### Stand der Technik

Über quantitative Messungen von Analyten in Ausatemluft können bestimmte Atemwegserkrankungen erkannt und überwacht werden. Beispielsweise kann über die Bestimmung der Stickstoffmonoxidkonzentration in ausgeatmeter Luft ein Maß für die Entzündung der Lunge bei Asthma abgeschätzt werden.

Die Bestimmung der Stickstoffmonoxidkonzentration kann dabei mit einer in EP 1384069 B1 offenbarten Vorrichtung über die Konversion von Stickstoffmonoxid zu Stickstoffdioxid mit nachfolgender Messung der Stickstoffdioxidkonzentration mithilfe eines feldeffektransistorbasierten Gassensors in der Vorrichtung erfolgen. Das Mundstück einer solchen Vorrichtung, über welches der Benutzer in die Vorrichtung hinein ausatmet, kann aus hygienischen Gründen als austauschbares Wegwerfprodukt ausgeführt sein. Insbesondere bei einem Einsatz der Vorrichtung in Arztpraxen oder Krankenhäusern sollte sichergestellt sein, dass dabei keine Mehrfachnutzung des Mundstückes durch verschiedene Benutzer erfolgt

Ein Mundstück mit einer den Gaspfad blockierenden Folie wird in US 9643186 B1 offenbart.

### Offenbarung der Erfindung

### Vorteile der Erfindung

Gegenstand der Erfindung ist ein System zur Messung von Analyten in Ausatemluft, wobei das System ein Mundstück und eine Vorrichtung zur Messung von Analyten in Ausatemluft umfasst, mit den Merkmalen des Anspruchs 1.

Die Vorrichtung des erfindungsgemäßen Systems kann beispielsweise auf einer in EP 1384069 B1 offenbarten Vorrichtung basieren. Bevorzugt ist der Drucksensor in einem Pfad der Vorrichtung angeordnet, welcher mit dem Gaspfad des verbundenen Mundstücks bei zerstörter Folie in fluidischer Verbindung steht. Wenn die Vorrichtung die oben genannte Pumpe aufweist, kann der Drucksensor derart in der Vorrichtung angeordnet und eingerichtet sein, dass er einen zwischen der Pumpe und der Folie des mit der Vorrichtung verbundenen Mundstücks herrschenden Druck über eine vorgegebene Zeit messen kann. Durch die Vorgabe von Bedingungen zum Drucklauf für eine Freigabe einer nachfolgenden Messung kann vorteilhafterweise festgestellt werden, ob die Folie im Mundstück noch intakt ist und den Gaspfad im Mundstück blockiert. Eine Zerstörung der Folie kann wie oben beschrieben vorteilhafterweise durch einen Benutzer des Mundstücks erfolgen. Alternativ oder unterstützend kann die Zerstörung durch ein Pumpen der Pumpe erfolgen. Eine schnelle, abrupte Druckänderung entspricht dabei einem Reißen der Folie. Wenn die Folie gerissen ist, kommt es zu einer vergleichbar langsameren Druckänderung. Somit kann die Vorrichtung vorteilhafterweise selbständig feststellen, vorzugsweise durch eine Auswertung in der oben genannten Steuereinrichtung, ob ein noch nicht bestimmungsgemäß benutztes Mundstück mit der Vorrichtung verbunden ist und in diesem Fall eine Benutzung der Vorrichtung für eine nachfolgende Messung freigeben.

Die Vorrichtung des erfindungsgemäßen Systems, insbesondere die oben genannte Steuereinrichtung, ist vorzugsweise derart eingerichtet, dass eine der vorgegebenen Bedingungen erfüllt ist, wenn ein vorgegebener Druck im Zuge des gemessenen Druckverlaufs erreicht wurde. Alternativ kann die Vorrichtung beziehungsweise die Steuereinrichtung derart eingerichtet sein, dass eine der vorgegebenen Bedingungen erfüllt ist, wenn ein vorgegebener Druckverlauf, ein vorgegebener Druckgradient oder eine vorgegebene Druckänderungsrate über den Drucksensor festgestellt wird. Dabei kann entweder ein Druck oberhalb eines Umgebungsdrucks, beispielsweise oberhalb des Standarddrucks auf Meereshöhe oder ein Druck unterhalb eines Umgebungsdrucks, beispielsweise unterhalb des Standarddrucks auf Meereshöhe, vorgegeben werden, vorzugsweise wenn die Pumpe eingerichtet ist, in Richtung der Folie zu pumpen beziehungsweise aus der Richtung der Folie zu saugen. Für den vorgegebenen Druck wird dabei bevorzugt ein Druckwert gewählt, welcher messbarüber beziehungsweise unter einem Umgebungsdruck liegt, beispielsweise zwischen 10 und 30 mbar über beziehungsweise unter dem Umgebungsdruck. Somit kann vorteilhafterweise festgestellt werden, ob der Gaspfad blockiert ist. und damit auf eine noch nicht bestimmungsgemäße Verwendung des Mundstücks rückgeschlossen werden.

In einer besonders vorteilhaften Ausgestaltung des erfindungsgemäßen Systems ist eine Pumpleistung der Pumpe der Vorrichtung abgestimmt auf eine vorgegebenen Reißfestigkeit der Folie des Mundstücks dazu eingerichtet, durch ein Ansaugen von Fluid, insbesondere Luft, aus einer Richtung von der Folie des Mundstücks oder durch ein Pumpen von Fluid, insbesondere Luft, in eine Richtung zur Folie des Mundstücks, ein Reißen der Folie zu bewirken. In der ersten Variante ist die Pumpe eingerichtet, Fluid, insbesondere Luft, aus der Richtung der Folie anzusaugen, um die Folie zum Reißen zu bringen. Dadurch wird zwischen der Folie und der Pumpe ein Unterdruck erzeugt. In der zweiten Variante ist die Pumpe eingerichtet, Fluid, insbesondere Luft, in Richtung der Folie des Mundstücks zu pumpen, um die Folie zum Reißen zu bringen. Anstatt einer Erzeugung eines Unterdrucks zwischen der Pumpe und der Folie wird hierbei ein Überdruck zwischen der Pumpe und der Folie erzeugt. Beide Varianten haben den Vorteil, dass der Benutzer das Reißen der Folie für eine bestimmungsgemäße Verwendung des Systems nicht selbst vornehmen muss. Hingegen kann die Vorrichtung kann das Reißen der Folie bewirken.

Die Vorrichtung, insbesondere die Steuereinrichtung, ist dabei vorzugsweise derart eingerichtet, dass die Pumpe für ein Reißen der Folie im mit der Vorrichtung verbundenen Mundstück auf eine vorgegebene Mindestleistung angesteuert wird. Die Mindestleistung kann dabei vorzugsweise auf eine Reißfestigkeit der Folie im Mundstück abgestimmt sein, so dass bei einem Ansteuern der Pumpe auf die Mindestleistung bei bestimmungsgemäßen Gebrauchs des Systems vorteilhafterweise ein Reißen der Folie bewirkt wird.

Insbesondere kann die vorgegebene Mindestleistung über einen zu erreichenden Druck auf eine Reißfestigkeit der Folie, gegebenenfalls unter Berücksichtigung der oben beschriebenen Sollbruchstelle, abgestimmt sein. Vorzugsweise kann die Vorrichtung dabei eingerichtet sein, die vorgegebene Mindestleistung kontinuierlich zu erhöhen, bis der Drucksensor eine vorgegebene Druckänderung misst, beispielsweise eine Druckänderung zwischen 10 und 30 mbar, insbesondere zwischen 20 und 25 mbar. Ein solche Druckänderung erfolgt nach dem Reißen der Folie durch den damit verbundenen Druckausgleich, so dass ein erfolgreiches Reißen der Folie vorteilhafterweise durch die Vorrichtung selbst erkannt werden kann, insbesondere über die dafür eingerichtete Steuereinrichtung.

Gemäß der oben genannten ersten Variante kann die Vorrichtung vorzugsweise dabei eingerichtet sein, die vorgegebene Mindestleistung der Pumpe kontinuierlich zu erhöhen, bis der Drucksensor einen vorgegebenen Druckabfall misst. Ein solcher Druckabfall erfolgt nach dem Reißen der Folie durch die damit verbundene Verringerung des zwischen der Folie und der Pumpe herrschenden Überdrucks. Gemäß der oben genannten zweiten Variante kann die Vorrichtung vorzugsweise dabei eingerichtet sein, die vorgegebene Mindestleistung kontinuierlich zu erhöhen, bis der Drucksensor einen vorgegebenen Druckanstieg misst. Der Druckanstieg resultiert hierbei durch eine Verringerung des zwischen der Folie und der Pumpe herrschenden Unterdrucks nach dem Reißen der Folie. Eine solche kontinuierliche Erhöhung der vorgegebenen Mindestleistung der Pumpe hat den Vorteil, unterschiedlich reißfeste Folien zuverlässig zerstört werden können und somit eine Abstimmung der Mindestleistung auf eine gegebenen Reißfestigkeit einer Folie vorteilhafterweise nicht erforderlich ist.

Gegenstand der Erfindung ist ferner ein Verfahren zur Messung von Analyten in Ausatemluft, mit den Merkmalen des Anspruchs 10. In einem ersten Schritt wird ein Mundstück mit einer Vorrichtung zur Messung von Analyten in Ausatemluft verbunden, wobei das Mundstück einen Gaspfad für eine Leitung von Ausatemluft zur Vorrichtung aufweist, wobei das Mundstück eine Folie umfasst, wobei die Folie derart angeordnet ist, dass sie die Leitung der Ausatemluft durch den Gaspfad blockiert. In einem zweiten Schritt erfolgt eine Messung eines Druckverlaufs durch einen Drucksensor in der Vorrichtung. In einem dritten Schritt erfolgt eine Freigabe der Vorrichtung für eine Messung vorgegebener Analyten in der Ausatemluft, wenn der gemessene Druckverlauf ein oder mehrere vorgegebene Bedingungen erfüllt. Diese Bedingungen entsprechen dabei einem Ende der Blockade aufgrund der gerissenen Folie.

Zu den Vorteilen des erfindungsgemäßen Verfahrens und seiner untenstehenden Weiterbildungen wird auf die korrespondierenden obengenannten Vorteile des offenbarten erfindungsgemäßen Systems verwiesen.

In einer vorteilhaften Weiterbildung des Verfahrens wird eine Pumpe der Vorrichtung für ein Reißen der Folie angesteuert. Dabei wird die Pumpe insbesondere zum Pumpen von Ausatemluft aus dem oder über das verbundene Mundstück angesteuert.

In einer weiteren vorteilhaften Weiterbildung des Verfahrens ist eine der vorgegebenen Bedingungen erfüllt, wenn ein vorgegebener Druck im Zuge des gemessenen Druckverlaufs erreicht wird.

Gemäß einer vorteilhaften Ausgestaltung des Verfahrens wird die Pumpe für ein Reißen der Folie im mit der Vorrichtung verbundenen Mundstück auf eine vorgegebene Mindestleistung angesteuert.

Bevorzugt wird dabei die vorgegebene Mindestleistung kontinuierlich erhöht wird, bis der Drucksensor eine vorgegebene Druckänderung misst.

### Kurze Beschreibung der Zeichnungen

Ausführungsbeispiele der Erfindung sind in den Zeichnungen schematisch dargestellt und in der nachfolgenden Beschreibung näher erläutert. Für die in den verschiedenen Figuren dargestellten und ähnlich wirkenden Elemente werden gleiche Bezugszeichen verwendet, wobei auf eine wiederholte Beschreibung der Elemente verzichtet wird.

Es zeigen
- Figur 1: ein Ausführungsbeispiel des Mundstücks,
- Figuren 2a,: b ein Ausführungsbeispiel des erfindungsgemäßen Systems,
- Figur 3: ein Diagramm eines Druckverlaufs bei Benutzung eines Ausführungsbeispiels des erfindungsgemäßen Systems und
- Figur 4: ein Ausführungsbeispiel der erfindungsgemäßen Verfahrens.

### Ausführungsformen der Erfindung

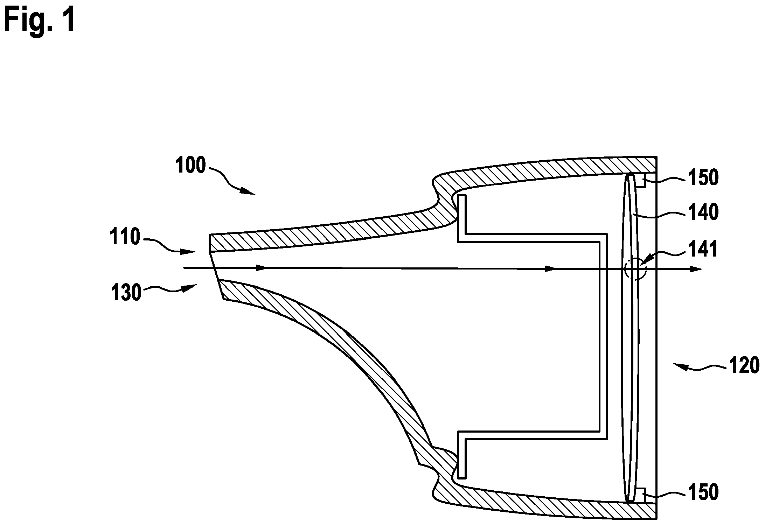

Figur 1 zeigt ein Ausführungsbeispiel des Mundstücks 100 für eine Vorrichtung zur Messung von Analyten in Ausatemluft, auch Gerät zur Atemgasanalyse genannt. Das Mundstück 100 weist eine erste Öffnung 110 als Einlassöffnung auf, welche mit einer Lippe eines Benutzers für ein Ausatmen in das Mundstück 100 kontaktiert werden kann. Beispielsweise ist die erste Öffnung 110 durch einen Teil des Mundstücks 100 begrenzt, welcher, wie in Figur 1 gezeigt, der Form eines Endes einer Schnabelflöte gleicht. Das Mundstück 100 umfasst auch eine zweite Öffnung 120, welche für eine Verbindung mit der Vorrichtung vorgesehen ist. Zwischen der ersten Öffnung 110 und der zweiten Öffnung 120 umfasst das Mundstück 100 einen Gaspfad 130 für eine Leitung von Ausatemluft zur Vorrichtung. Ferner umfasst das Mundstück 100 eine Folie 140, wobei die Folie 140 derart angeordnet ist, dass sie die Leitung der Ausatemluft durch den Gaspfad 130 blockiert. Bei der Folie 140 kann es sich beispielsweise um eine Kunststofffolie, insbesondere mit Polyethylen und/oder mit Polypropylen handeln, welche beispielsweise eine Dicke zwischen 0,01 und 0,1 Millimeter aufweist. Durch die Wahl des Material und der Dicke der Folie 140 kann abhängig vom Durchmesser des Gaspfads 130 festgelegt werden, wie hoch ein auf die Folie 140 wirkender Überdruck oder Unterdruck ausfallen muss, um die Folie 140 zum Reißen zu bringen, beispielsweise durch ein Einblasen eines Benutzers in die Öffnung 110 des Mundstücks 100. Insbesondere kann dadurch ein Mindestdruckunterschied zwischen einem ersten Druck auf einer Seite der Folie 140 und einem zweiten Druck auf der anderen Seite der Folie 140 als Grenzwert für ein Reißen der Folie eingestellt werden. Beispielsweise kann der Mindestdruckunterschied zwischen 10 und 25 Millibar betragen. Das Gehäuse des Mundstücks 100 kann ebenfalls aus Kunststoff gefertigt sein.

Um die Zerstörung der Folie 140 zu erleichtern, einen wohldefinierten Riss und/oder eine wohldefinierten Mindestdruck zu gewährleisten, kann die Folie 140 eine Sollbruchstelle 141 aufweisen, beispielsweise in Form eines Materialabtrags für eine Verdünnung der Dicke der Folie 140 am Ort der Sollbruchstelle. Wie in Figur 1 dargestellt, ist die Sollbruchstelle 141 vorteilhafterweise in einem Bereich der Folie 140 angeordnet, welcher den Gaspfad 130 blockiert, so dass bei einem Reißen der Folie 140 der Gaspfad 130 nicht mehr durch die Folie 140 blockiert wird.

Die Folie 140 kann an einem Vorsprung 150 des Mundstücks 100 befestigt sein, beispielsweise über einen Klebstoff. Der Vorsprung 150 kann dabei die Form einer den Gaspfad 130 umgebenden Schleife aufweisen, insbesondere die Form eines Ringes.

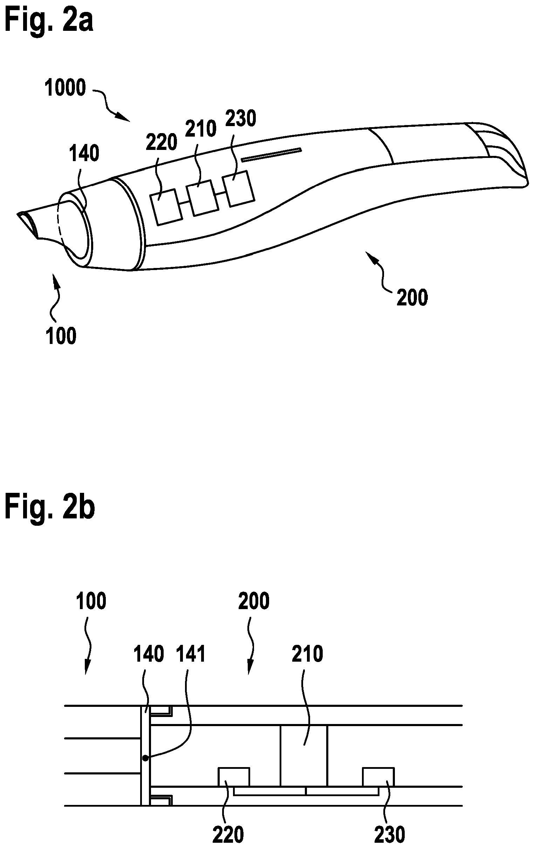

Figur 2a zeigt ein Ausführungsbeispiel des erfindungsgemäßen Systems 1000 zur Messung von Analyten in Ausatemluft umfassend ein Ausführungsbeispiel eines Mundstücks 100 und eine Vorrichtung 200 zur Messung von Analyten in Ausatemluft. Bei dem Mundstück 100 kann es sich beispielsweise um die oben beschriebene Ausführungsform zu Figur 1 handeln. Das Ausführungsbeispiel der Vorrichtung 200 kann beispielsweise auf einer in EP 1384069 B1 offenbarten Vorrichtung basieren.

Das Mundstück 100 ist mit der Vorrichtung 200 lösbar verbindbar, beispielsweise über eine formschlüssige Verbindung, beispielsweise eine Clipverbindung. Die Vorrichtung 200 umfasst eine Pumpe 210 zum Ansaugen von Ausatemluft über das verbundene Mundstück 100 und einen Drucksensor 220. Die Vorrichtung 200 ist dazu eingerichtet, beispielsweise über eine Einrichtung eines Steuergeräts 230 in der Vorrichtung 200, die Vorrichtung 200 für eine Messung vorgegebener Analyten in der Ausatemluft freizugeben, wenn ein über den Drucksensor 220 gemessener Druckverlauf ein oder mehrere vorgegebene Bedingungen erfüllt.

Figur 2b zeigt einen schematischen Ausschnitt des Mundstücks 100 und der mit dem Mundstück 100 verbundenen Vorrichtung 200 zum Ausführungsbeispiel aus Figur 2a. Wie aus Figur 2b ersichtlich, ist der Drucksensor 220 zwischen der Pumpe 210 und der Folie 140 des mit der Vorrichtung 200 verbundenen Mundstücks 100 angeordnet und kann somit einen Druck in dem Bereich zwischen Folie 140 und Pumpe 210 messen. Eine Steuereinrichtung 230, insbesondere ein Steuergerät 230, ist mit dem Drucksensor 220 für einen Empfang von Sensordaten und mit der Pumpe 210 für eine Ansteuerung der Pumpe 210 verbunden. Wie oben ausgeführt, ist die Steuereinrichtung 230 eingerichtet, die Vorrichtung 200 für eine Messung vorgegebener Analyten in der Ausatemluft freizugeben, wenn ein über den Drucksensor 220 gemessener Druckverlauf ein oder mehrere vorgegebene Bedingungen erfüllt. Beispielsweise kann die Steuereinrichtung 230 und somit die Vorrichtung 200 derart eingerichtet sein, dass eine der vorgegebenen Bedingungen erfüllt ist, wenn ein vorgegebener Unterdruck oder Mindestdruck im Zuge des vom Sensor 220 gemessenen Druckverlaufs erreicht oder unterschritten wird. Bei einem angeschlossenen Mundstück 100 kann ein solcher Mindestdruck nur erreicht werden, wenn der Gaspfad 130 blockiert ist. Somit kann auf eine intakte oder zerstörte Folie 140 rückgeschlossen werden.

Die Pumpe 230 kann auch eingerichtet sein, in Richtung der Folie 140 zu pumpen. In diesem Fall kann die Vorrichtung 200, insbesondere die Steuereinrichtung 230, eingerichtet sein, dass eine der vorgegebenen Bedingungen erfüllt ist, wenn ein vorgegebener Überdruck oder Maximaldruck im Zuge des vom Sensor 220 gemessenen Druckverlaufs erreicht oder überschritten wird. Wie im Falle eines von der Pumpe 210 erzeugten Unterdrucks kann ein solcher Überdruck oder Maximaldruck nur erreicht werden, wenn der Gaspfad 130 blockiert ist. Somit kann auf diese alternative Weise auf eine intakte oder zerstörte Folie 140 rückgeschlossen werden.

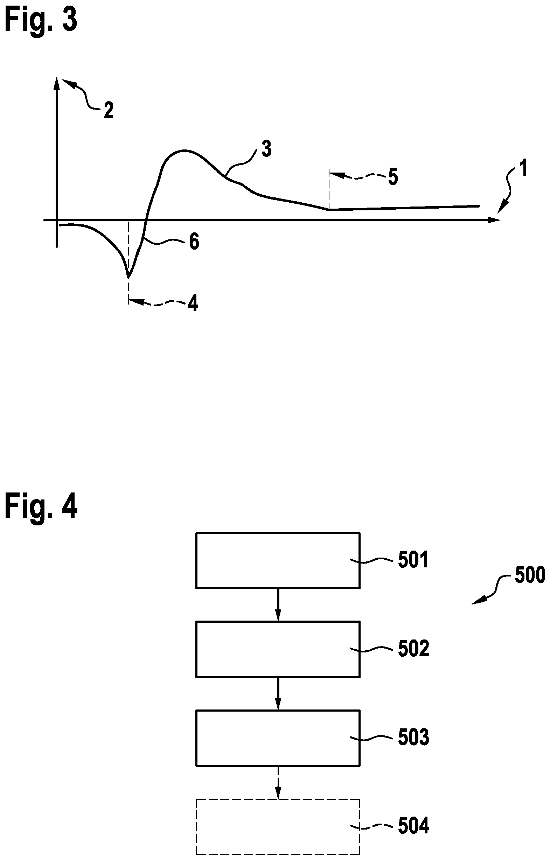

Ein Reißen der Folie 140 kann entweder über ein Ausatmen oder Einblasen des Benutzers in das Mundstück 100, über eine Ansteuerung der Pumpe 210 oder über eine Kombination der beiden Varianten erfolgen. Beispielsweise kann die Steuereinrichtung 230 und somit die Vorrichtung 200 eingerichtet sein, die Pumpe 210 für ein Reißen der Folie 140 auf eine vorgegebene Mindestleistung anzusteuern. Dabei ist die vorgegebene Mindestleistung abhängig von der Reißfestigkeit der Folie 140 aufgrund des Materials, der Dicke und gegebenenfalls der Sollbruchstelle 141 derart eingestellt, dass eine Reißen der Folie 140 bei Erreichen der Mindestleistung aufgrund der damit verbundenen Pumpleistung und des daraus folgenden Drucks auf die Folie 140 erfolgt ist. Der Druck auf die Folie 140 kann dabei durch die Erzeugung eines Unterdrucks oder eines Überdrucks in dem Bereich zwischen der Folie 140 und der Pumpe 210 bewirkt werden. Figur 3 zeigt einen beispielhaften Druckverlauf 3 in dem vom Drucksensor 220 erfassten Bereich zwischen der Folie 140 und der Pumpe 210 bei einer Erzeugung eines Unterdrucks zwischen der Folie 140 und der Pumpe 210. Die horizontale Achse 1 des Diagramms in Figur 3 zeigt dabei die Zeit und die vertikale Achse 2 den Druck in dem Bereich in jeweils willkürlichen Einheiten. Zu Beginn der Tätigkeit der Pumpe 210 wird von dem Drucksensor 220 ein Druckabfall bis zu einem ersten Zeitpunkt 4 erfasst. Zum ersten Zeitpunkt 4 reißt die Folie und es kommt nach einem abrupten Druckanstieg 6 zu einem kurzzeitigen Überdruck 3 in dem Bereich, bis sich ab dem zweiten Zeitpunkt 5 ein vergleichsweise stabiler Druck in dem Bereich einstellt. Für die Darstellung eines Druckverlaufs im Fall der Erzeugung eines Überdrucks zwischen der Folie 140 und der Pumpe 210 muss das Diagramm aus Figur 3 entlang der horizontalen Achse 1 gespiegelt werden.

Alternativ zu einer fixen vorgegebenen Mindestleistung kann die Vorrichtung 200, insbesondere die Steuereinrichtung 230, derart eingerichtet sein, dass die vorgegebene Mindestleistung kontinuierlich erhöht wird, bis der Drucksensor 220 eine vorgegebene Druckänderung misst. Wie oben beschrieben und in Figur 3 dargestellt, erfolgt im Falle der Erzeugung eines Unterdrucks zwischen der Folie 140 und der Pumpe 210 eine solche Druckänderung in Form eines Druckanstiegs 6 nach einem Reißen der Folie 140.

Figur 4 zeigt ein Ablaufdiagramm zu einem Ausführungsbeispiel des erfindungsgemäßen Verfahrens 500, beispielsweise ausgeführt mit einem Mundstück 100 und einer Vorrichtung 200 gemäß den oben beschriebenen Ausführungsbeispielen. In einem ersten Schritt 501 wird das Mundstück 100 mit der Vorrichtung 200 zur Messung von Analyten in Ausatemluft verbunden, wobei das Mundstück 100 einen 130 Gaspfad für eine Leitung von Ausatemluft zur Vorrichtung aufweist und wobei das Mundstück 100 eine Folie 140 umfasst, wobei die Folie 140 derart angeordnet ist, dass sie die Leitung der Ausatemluft durch den Gaspfad 130 blockiert. In einem zweiten Schritt 502 wird eine Pumpe 210 der Vorrichtung 200 zum Pumpen von Luft über das verbundene Mundstück 100, insbesondere Ausatemluft, angesteuert und ein Druckverlaufs durch einen Drucksensor 220 der Vorrichtung 200 gemessen. Alternativ oder zusätzlich kann ein Benutzer in die Öffnung 110 des Mundstücks 100 hineinblasen, um die Folie 140 zum Reißen zu bringen, vorzugsweise unterstützt durch die Pumpe 210. In einem dritten Schritt 503 wird die Vorrichtung 200 für eine Messung vorgegebener Analyten in der Ausatemluft freigegeben, wenn der gemessene Druckverlauf ein oder mehrere vorgegebene Bedingungen erfüllt. Eine der vorgegebenen Bedingungen kann dabei erfüllt sein, wenn ein vorgegebener Druck im Zuge des gemessenen Druckverlaufs erreicht wurde. Für ein Reißen der Folie 140 kann die Pumpe 210 auf eine vorgegebene Mindestleistung angesteuert werden. Dies kann im Zuge des zweiten Schritts 502 oder in einem vierten Schritt nach einer Freigabe der Vorrichtung 200 für die Messung erfolgen. Dabei kann ferner die vorgegebene Mindestleistung kontinuierlich erhöht werden, bis der Drucksensor 220 einen vorgegebenen Druckanstieg aufgrund des Reißens der Folie 140 misst.

## Patentansprüche

1. System (1000) zur Messung von Analyten in Ausatemluft, umfassend ein Mundstück (100) und eine Vorrichtung (200) zur Messung von Analyten in Ausatemluft, wobei das Mundstück (100) einen Gaspfad (130) für eine Leitung von Ausatemluft zur Vorrichtung (200) aufweist, wobei das Mundstück (100) eine Folie (140) umfasst, wobei die Folie (140) derart angeordnet ist, dass sie die Leitung der Ausatemluft durch den Gaspfad (130) blockiert, wobei das Mundstück (100) mit der Vorrichtung (200) lösbar verbindbar ist, **dadurch gekennzeichnet dass** die Vorrichtung (200) einen Drucksensor (220) umfasst und dazu eingerichtet ist, die Vorrichtung (200) für eine Messung vorgegebener Analyten in der Ausatemluft freizugeben, wenn ein über den Drucksensor (220) gemessener Druckverlauf ein oder mehrere vorgegebene Bedingungen erfüllt.

2. System (1000) nach einem der vorhergehenden Ansprüche, wobei die Folie (140) innerhalb des Mundstücks (100) angeordnet ist, insbesondere in einem Innenraum des Mundstücks oder im Gaspfad (130).

3. System (1000) nach einem der vorhergehenden Ansprüche, wobei die Folie (140) eine Sollbruchstelle (141) aufweist, vorzugsweise in einem Bereich der Folie (140), welcher den Gaspfad (130) blockiert.

4. System (1000) nach einem der vorhergehenden Ansprüche, wobei die Folie (140) an einem Vorsprung (150) im Mundstück (100) befestigt, insbesondere angeklebt, ist.

5. System (1000) nach Anspruch 4, wobei die Vorrichtung (200) derart eingerichtet ist, dass eine der vorgegebenen Bedingungen erfüllt ist, wenn ein vorgegebener Druck im Zuge des gemessenen Druckverlaufs erreicht wurde.

6. System (1000) nach Anspruch 4 oder 5, wobei die Vorrichtung (200) eine Pumpe (210) zum Pumpen von Luft aus dem oder über das verbundene Mundstück (100) umfasst.

7. System (1000) nach Anspruch 6, wobei eine Pumpleistung der Pumpe (210) der Vorrichtung (200) abgestimmt auf eine vorgegebenen Reißfestigkeit der Folie (140) des Mundstücks (100) dazu eingerichtet ist, durch ein Ansaugen von Fluid, insbesondere Luft, aus einer Richtung zur Folie (140) des Mundstücks (100) oder durch ein Pumpen von Fluid, insbesondere Luft, in eine Richtung zur Folie (140) des Mundstücks (100), ein Reißen der Folie (140) zu bewirken.

8. System (1000) nach Anspruch 6 oder 7, wobei die Vorrichtung (200) derart eingerichtet ist, dass die Pumpe (210) für ein Reißen der Folie (140) im mit der Vorrichtung (200) verbundenen Mundstück (100) auf eine vorgegebene Mindestleistung angesteuert wird.

9. System (1000) nach Anspruch 8, wobei die Vorrichtung (200) eingerichtet ist, die vorgegebene Mindestleistung für ein Reißen der Folie (140) kontinuierlich zu erhöhen, bis der Drucksensor (220) eine vorgegebene Druckänderung misst.

10. Verfahren (200) zur Freigabe einer Messung von Analyten in Ausatemluft, umfassend die Schritte:
• Verbinden eines Mundstücks (100) mit einer Vorrichtung (200) zur Messung von Analyten in Ausatemluft, wobei das Mundstück (100) einen Gaspfad (130) für eine Leitung von Ausatemluft zur Vorrichtung (200) aufweist, wobei das Mundstück (100) eine Folie (140) umfasst, wobei die Folie (140) derart angeordnet ist, dass sie die Leitung der Ausatemluft durch den Gaspfad (130) blockiert,
**gekennzeichnet durch**
• Messung eines Druckverlaufs durch einen Drucksensor (220) in der Vorrichtung (200)
• Freigabe der Vorrichtung (200) für eine Messung vorgegebener Analyten in der Ausatemluft, wenn der gemessene Druckverlauf ein oder mehrere vorgegebene Bedingungen erfüllt, welche eine Ende der Blockade durch die Folie (140) entsprechen

11. Verfahren (200) nach Anspruch 10, wobei eine Pumpe (210) der Vorrichtung (200), insbesondere zum Pumpen von Luft aus dem oder über das verbundene Mundstück (100), für ein Reißen der Folie (140) angesteuert wird.

12. Verfahren (200) nach Anspruch 10 oder 11, wobei eine der vorgegebenen Bedingungen erfüllt ist, wenn ein vorgegebener Druck im Zuge des gemessenen Druckverlaufs erreicht wird.

13. Verfahren (200) nach einem der Ansprüche 11 oder 12, wobei die Pumpe (210) für ein Reißen der Folie (140) im mit der Vorrichtung (200) verbundenen Mundstück (100) auf eine vorgegebene Mindestleistung angesteuert wird.

14. Verfahren (200) nach Anspruch 13, wobei die vorgegebene Mindestleistung kontinuierlich erhöht wird, bis der Drucksensor (220) eine vorgegebene Druckänderung misst.

## Claims

1. System (1000) for the measurement of analytes in exhaled air, comprising a mouthpiece (100) and a device (200) for the measurement of analytes in exhaled air, the mouthpiece (100) having a gas path (130) for a conduction of exhaled air to the device (200), the mouthpiece (100) comprising a film (140), the film (140) being arranged such that it blocks the conduction of the exhaled air through the gas path (130), the mouthpiece (100) being separably connectable to the device (200), **characterized in that** the device (200) comprises a pressure sensor (220) and is configured to enable the device (200) for a measurement of specified analytes in the exhaled air when a pressure profile measured via the pressure sensor (220) meets one or more specified conditions.

2. System (1000) according to any of the preceding claims, wherein the film (140) is arranged within the mouthpiece (100), especially in an internal space of the mouthpiece or in the gas path (130).

3. System (1000) according to any of the preceding claims, wherein the film (140) has a predetermined breaking point (141), preferably in a region of the film (140) that is blocking the gas path (130).

4. System (1000) according to any of the preceding claims, wherein the film (140) is fixed, especially adhesively bonded, to a protrusion (150) in the mouthpiece (100).

5. System (1000) according to Claim 4, wherein the device (200) is configured such that one of the specified conditions is met when a specified pressure has been reached in the course of the measured pressure profile.

6. System (1000) according to Claim 4 or 5, wherein the device (200) comprises a pump (210) for the pumping of air from the or across the connected mouthpiece (100).

7. System (1000) according to Claim 6, wherein a pump output of the pump (210) of the device (200), matched to a specified tear strength of the film (140) of the mouthpiece (100), is configured to bring about a tearing of the film (140) by means of a suction of fluid, especially air, from a direction to the film (140) of the mouthpiece (100) or by means of a pumping of fluid, especially air, in a direction to the film (140) of the mouthpiece (100).

8. System (1000) according to Claim 6 or 7, wherein the device (200) is configured such that the pump (210) is actuated at a specified minimum output for a tearing of the film (140) in the mouthpiece (100) connected to the device (200).

9. System (1000) according to Claim 8, wherein the device (200) is configured to continuously increase the specified minimum output for a tearing of the film (140) until the pressure sensor (220) measures a specified pressure change.

10. Method (200) for enabling a measurement of analytes in exhaled air, comprising the steps of:
• connecting a mouthpiece (100) to a device (200) for the measurement of analytes in exhaled air, the mouthpiece (100) having a gas path (130) for a conduction of exhaled air to the device (200), the mouthpiece (100) comprising a film (140), the film (140) being arranged such that it blocks the conduction of the exhaled air through the gas path (130),
**characterized by**
• measuring a pressure profile by means of a pressure sensor (220) in the device (200)
• enabling the device (200) for a measurement of specified analytes in the exhaled air when the measured pressure profile meets one or more specified conditions which correspond to an end of the blockade by the film (140)

11. Method (200) according to Claim 10, wherein a pump (210) of the device (200), especially for the pumping of air from the or across the connected mouthpiece (100), is actuated for a tearing of the film (140).

12. Method (200) according to Claim 10 or 11, wherein one of the specified conditions is met when a specified pressure is reached in the course of the measured pressure profile.

13. Method (200) according to either of Claims 11 and 12, wherein the pump (210) is actuated at a specified minimum output for a tearing of the film (140) in the mouthpiece (100) connected to the device (200).

14. Method (200) according to Claim 13, wherein the specified minimum output is continuously increased until the pressure sensor (220) measures a specified pressure change.

## Revendications

1. Système (1000) permettant de mesurer des analytes dans l'air expiré, comprenant un embout buccal (100) et un dispositif (200) permettant de mesurer des analytes dans l'air expiré, l'embout buccal (100) présentant un trajet de gaz (130) pour guider l'air expiré vers le dispositif (200), l'embout buccal (100) comprenant un film (140), le film (140) étant disposé de telle sorte qu'il bloque le guidage de l'air expiré sur le trajet de gaz (130), l'embout buccal (100) pouvant être raccordé au dispositif (200) de manière amovible,
**caractérisé en ce que** le dispositif (200) comprend un capteur de pression (220) et est aménagé pour libérer le dispositif (200) pour une mesure d'analytes prédéfinis dans l'air expiré si une courbe de pression mesurée par l'intermédiaire du capteur de pression (220) remplit une ou plusieurs conditions prédéfinies.

2. Système (1000) selon l'une quelconque des revendications précédentes, dans lequel le film (140) est disposé à l'intérieur de l'embout buccal (100), en particulier dans un espace intérieur de l'embout buccal ou sur le trajet de gaz (130).

3. Système (1000) selon l'une quelconque des revendications précédentes, dans lequel le film (140) présente un point destiné à la rupture (141), de préférence dans une zone du film (140) bloquant le trajet de gaz (130).

4. Système (1000) selon l'une quelconque des revendications précédentes, dans lequel le film (140) est fixé, en particulier collé, à une saillie (150) dans l'embout buccal (100).

5. Système (1000) selon la revendication 4, dans lequel le dispositif (200) est aménagé de telle sorte que l'une des conditions prédéfinies est remplie si une pression prédéfinie est atteinte au cours de la courbe de pression mesurée.

6. Système (1000) selon la revendication 4 ou 5, dans lequel le dispositif (200) comprend une pompe (210) permettant de pomper de l'air à partir ou par l'intermédiaire de l'embout buccal raccordé (100).

7. Système (1000) selon la revendication 6, dans lequel une puissance de pompage de la pompe (210) du dispositif (200), adaptée à une résistance à la rupture prédéfinie du film (140) de l'embout buccal (100), est aménagée pour provoquer une rupture du film (140) par une aspiration de fluide, en particulier d'air, à partir d'une direction vers le film (140) de l'embout buccal (100) ou par un pompage de fluide, en particulier d'air, dans une direction vers le film (140) de l'embout buccal (100).

8. Système (1000) selon la revendication 6 ou 7, dans lequel le dispositif (200) est aménagé de telle sorte que la pompe (210) est pilotée avec une puissance minimale prédéfinie en vue d'une rupture du film (140) dans l'embout buccal (100) raccordé au dispositif (200).

9. Système (1000) selon la revendication 8, dans lequel le dispositif (200) est aménagé pour augmenter en continu la puissance minimale prédéfinie en vue d'une rupture du film (140) jusqu'à ce que le capteur de pression (220) mesure une variation de pression prédéfinie.

10. Procédé (200) permettant de libérer une mesure d'analytes dans l'air expiré, comprenant les étapes consistant à :
• raccorder un embout buccal (100) à un dispositif (200) permettant de mesurer des analytes dans l'air expiré, l'embout buccal (100) présentant un trajet de gaz (130) pour guider l'air expiré vers le dispositif (200), l'embout buccal (100) comprenant un film (140), le film (140) étant disposé de telle sorte qu'il bloque le guidage de l'air expiré sur le trajet de gaz (130),
**caractérisé par**
• la mesure d'une courbe de pression par un capteur de pression (220) dans le dispositif (200),
• la libération du dispositif (200) pour une mesure d'analytes prédéfinis dans l'air expiré si la courbe de pression mesurée remplit une ou plusieurs conditions prédéfinies qui correspondent à la fin du blocage par le film (140).

11. Procédé (200) selon la revendication 10, dans lequel une pompe (210) du dispositif (200), en particulier pour pomper de l'air à partir ou par l'intermédiaire de l'embout buccal raccordé (100), est pilotée en vue d'une rupture du film (140).

12. Procédé (200) selon la revendication 10 ou 11, dans lequel l'une des conditions prédéfinies est remplie si une pression prédéfinie est atteinte au cours de la courbe de pression mesurée.

13. Procédé (200) selon l'une quelconque des revendications 11 et 12, dans lequel la pompe (210) est pilotée avec une puissance minimale prédéfinie en vue d'une rupture du film (140) dans l'embout buccal (100) raccordé au dispositif (200).

14. Procédé (200) selon la revendication 13, dans lequel la puissance minimale prédéfinie est augmentée en continu jusqu'à ce que le capteur de pression (220) mesure une variation de pression prédéfinie.
